# EUROPÄISCHE PATENTSCHRIFT

(11) **EP 2 594 283 B1**
(45) Veröffentlichungstag und Bekanntmachung des Hinweises auf die Patenterteilung: **23.11.2016**
(21) Anmeldenummer: 11807123.2
(22) Anmeldetag: 25.02.2011
(51) Int. Cl.: A61K 47/10, A61K 47/44, A61K 9/12, A61K 9/72, A61P 31/12, A61K 38/57

(54) **AEROSOLPRÄPARAT AUF APROTININBASIS ZUR BEHANDLUNG VON VIRUSINFEKTIONEN DER ATEMWEGE**
APROTININ-BASED AEROSOL PREPARATION FOR THE TREATMENT OF VIRAL RESPIRATORY INFECTIONS
PRÉPARATION AÉROSOL À BASE D'APROTININE POUR TRAITER LES INFECTIONS RESPIRATOIRES VIRALES

(30) Priorität: 15.07.2010 RU 2010129216
(43) Veröffentlichungstag der Anmeldung: 22.05.2013
(73) Patentinhaber: Torvald Ranta Företagsjuridik AB, 18275 Stocksund (SE)
(72) Erfinder: ZHIRNOV, Oleg Petrovich, Istrinsky raion Moskovskaya obl. 143350 (RU); KHANYKOV, Aleksandr Vladimirovich, Moscow 123458 (RU)
(74) Vertreter: Lemcke, Brommer & Partner Patentanwälte Partnerschaft mbB
(86) Internationale Anmeldenummer: PCT/RU2011/000104
(87) Internationale Veröffentlichungsnummer: WO 2012/008869

(56) Entgegenhaltungen:
- EP-A1- 0 563 389
- EP-A1- 0 563 389
- EP-A2- 0 372 777
- US-A- 5 618 786
- US-A- 5 618 786
- ZHIRNOV O P ET AL: "Aerosolized aprotinin is an effective drug against viral respiratory illness", ANTIINFECTIVE DRUGS AND CHEMOTHERAPY, XX, XX, Bd. 14, Nr. 3, 1. Januar 1996 (1996-01-01) , Seiten 209-216, XP008103403, ISSN: 0946-4832
- OVCHARENKO A V ET AL: "Aprotinin aerosol treatment of influenza and paramyxovirus bronchopneumonia of mice", ANTIVIRAL RESEARCH, ELSEVIER BV, NL, Bd. 23, Nr. 2, 1. Februar 1994 (1994-02-01), Seiten 107-118, XP023702748, ISSN: 0166-3542, DOI: 10.1016/0166-3542(94)90038-8 [gefunden am 1994-02-01]
- ZHIRNOV O P ET AL: "Aprotinin and similar protease inhibitors as drugs against influenza", ANTIVIRAL RESEARCH, ELSEVIER BV, NL, Bd. 92, Nr. 1, 18. Juli 2011 (2011-07-18), Seiten 27-36, XP028293106, ISSN: 0166-3542, DOI: 10.1016/J.ANTIVIRAL.2011.07.014 [gefunden am 2011-07-23]
- UCHENNA AGU REMIGIUS ET AL: "The lung as a route for systemic delivery of therapeutic proteins and peptides", RESPIRATORY RESEARCH, BIOMED CENTRAL LTD., LONDON, GB, Bd. 2, Nr. 4, 12. April 2001 (2001-04-12), Seiten 198-209, XP021021424, ISSN: 1465-9921, DOI: 10.1186/RR58
- SHOYELE S A ET AL: "Prospects of formulating proteins/peptides as aerosols for pulmonary drug delivery", INTERNATIONAL JOURNAL OF PHARMACEUTICS, ELSEVIER BV, NL, Bd. 314, Nr. 1, 11. Mai 2006 (2006-05-11), Seiten 1-8, XP025112932, ISSN: 0378-5173, DOI: 10.1016/J.IJPHARM.2006.02.014 [gefunden am 2006-05-11]
- OVCHINNIKOV YU. A.: 'Bioogranicheskaya khimiya' M., "PROSVESCHENIE" 1987, page 106
- GORDOX: 'Spravochnik Vidal. Lekarstvennye preparaty v Rossii' M., ASTRAFARMSERVIS 1997, page B-158

## Beschreibung

### Technisches Gebiet

Die vorliegende Erfindung betrifft die Medizin und bezieht sich insbesondere auf Heilmittelpräparate auf Grundlage von Aprotinin in Form eines Aerosols, angepasst an die Verwendung in einem gasdichten Behälter mit normierter Dosierung.

### Hintergrund der Technik

Zu den respiratorischen Infektionen der Virusätiologie mit dem schwersten Verlauf und ernsthaften Konsequenzen soll in erster Linie die Grippe gezählt werden. Die Sachlage verschlechtert sich dadurch, dass die moderne Medizin über eine begrenzte Palette an selektiv wirkenden Präparaten mit nachgewiesener klinischer Wirksamkeit und Sicherheit verfügt, die gegen respiratorische Viren (http:// www.med1001et.ru/pylmonologia "Bei respiratorischen Infektionen anwendbare Antiviruspräparate", *L.S. Stratschunski und S.N. Koslow,* Forschungsinstitut für antimikrobielle Chemotherapie, Smolensk) wirksam sind. Bei respiratorischen Infektionen verwendet man heutzutage zwei Gruppen von antigrippösen Präparaten, nämlich M₂-Kanal-Blocker (Amantadin, Rimantadin), und Neuroaminidase-Inhibitoren (Sanamivir, Oseltamivir) sowie Ribavirin, das gegen das respiratorische Sinzitialvirus wirksam ist.

Somit ist der wichtige Vorteil der Neuroaminidase-Inhibitoren gegenüber den M₂-Kanal-Bockern (Amandatin, Rimandatin) eine Wirksamkeit gegen zwei Virustypen A und B. Dank dem Vorhandensein der Präparate für deren orale Verabreichung und deren inhalatorische Anwendung bietet sich die Möglichkeit für eine individualisierte Therapie mit Rücksicht auf Besonderheiten eines konkreten Patienten.

Alle genannten Präparate mit Ausnahme von Ribaverin werden in Form von Tabletten oder Kapseln produziert und nach Verabreichung im Magen-Darm-Kanal eingesogen.

Ribavirin, das durch die Fa. ICN Pharmaceuticals (USA) unter der Handelsmarke Virazol in Flaschen zu je 6,0 g produziert wird, wird inhalatorisch mittels eines Nebulizers nur noch unter Bedingungen einer stationären medizinischen Einrichtung verabreicht. Vor der Prozedur wird das Füllgut einer Flasche in 300 g steriles Wasser für Injektionen (die Konzentration der verwendeten Lösung beläuft sich auf 20 mg pro ml) aufgelöst. Die Inhalation dauert 12 bis 18 Stunden tagtäglich, die Kur nimmt eine Zeit von 3 bis 7 Tagen in Anspruch.

Zur Zeit bestehen mehrere Typen von Inhalationssystemen, und zwar dosierte Aerosolinhalatoren, Pulverinhalatoren und Nebulizers, welche Heilmittelpräparate in gewünschter Konzentration an die oberen Luftwege und die Lungen unter geringer innertherapeutischer Beeinflussung heranreichen lassen.

Als am aussichtsreichsten gilt die Verpackung von als Aerosol vorliegenden Heilmittelpräparaten in Aerosolbehältern mit normierter Dosierung, welche Aerosolbehälter einfach, zuverlässig und preisgünstig sind. Aerosolbehälter für den medizinischen Einsatz enthalten ein unter Druck befindliches Treibgas (Arbeitsgas), einen Wirkstoff, einen oder mehrere Hilfsstoffe. Ein Heilmittelpräparat wird aus einem Behälter durch ein manuelles Betätigen eines Dosierventils freigesetzt, welches Heilmittelpräparat dann über das Ventil und Verbindungsrohre an die Auslaßöffnung unter Einwirkung eines im Behälter herrschenden Gasdruckes geführt wird.

Aerosolbehälter mit medizinischen Mitteln weisen eine Anzahl von Vorteilen gegenüber der üblichen Verpackung auf. Die Wirksamkeit eines Präparats erhöht sich dank einer Vergrößerung der damit bearbeiteten Oberfläche, wobei eine Keimfreiheit des Präparates gesichert wird, einer Verunreinigung mit Fremdstoffen begegnet wird und das ursprüngliche Verhalten des Präparates unabhängig von atmosphärischen Einflußgrößen erhalten bleibt. Bei der Verwendung von Aerosolbehältern bildet sich eine Wolke aus hyperfein dispergierten Teilchen eines Heilmittelpräparates, welche beim Einatmen durch einen Patienten in die Nasenoder Mundhöhle je nach dem Ansatzstück gelangen.

Für gasdichte Aerosolbehälter werden normalerweise nicht chlorierte Fluorwasserstoffe wie 1,1,1,2-Tetrafluorethan ("HFA-134a") und 1,1,1,2,3,3,3-Heptafluorpropan ("HFA-227ea) verwendet, die für die Gesundheit des Patienten am gefahrlosesten sind. Aerosolpräparate in Behältern liegen in Form von Lösungen oder Suspensionen vor.

Manche Präparate enthalten auch einen oder mehrere Hilfsstoffe für besondere Zwecke, wie ein Colösungsmittel oder ein Oberflächenaktivstoff (EP 0372777). Colösungsmittel sind Lösungsmittel, die als Dispersionsmittel (Wasser oder nichtwässrige Medien) benutzt werden und die für das Auflösen eines Wirkstoffes im Treibgas in therapeutisch wirksamer Menge verantwortlich sind. Normalerweise bedient man sich eines Colösungsmittels, das polarer ist als Treibgas. Als nichtwässrige Colösungsmittel kommen dabei Äthanol, Isopropylalkohol, Glykole, wie z. B. Propylenglykol, sowie Glyzerin, Polyoxyethylenalkohole in Frage. Suspensionen weisen in der Regel Oberflächenaktivstoffe zur Verhütung einer Agglomeration von Teilchen einer Aerosolzubereitung auf.

Es ist bereits ein Verfahren zur Behandlung von respiratorischen Virusinfektionen (RU-PS Nr. 2054180 vom 21.08.1991) bekannt, bei dem der Organismus mit aerosolartigen Proteinaseninhibitoren über den Respirationstrakt behandelt wird. Es wurde auch vorgeschlagen, Aprotinine und aprotininartige Inhibitoren als Wirkstoffe zu verwenden, die naturgemäße Eiweißverbindungen sind. Die Wirksamkeit der Aerosolpräparate wurde durch eine Einwirkung auf mit einem pathogenen Virus infizierte Mäuse getestet. Als Quelle für die Erzeugung eines Aprotininaerosols diente ein Ultraschallgenerator vom Typ Musson-1 oder ein pneumatischer, mit einem Luftfördersystem versehener Generator von der Art eines Ejektors. Bei einem System mit dem Ultraschallgenerator wurden bestimmte Wirkstoffmengen (7 bis 10 l/min.) mittels eines natürlichen Luftstromes einer Kammer zugeführt, indem darin ein Gas-Wasser-Eiweiß-Gemisch erzeugt wurde, das durch die Mäuse eingeatmet wurde. In 1 ml des Gemisches betrug der Gehalt des Eiweiß-Bestandteiles von 0,01 bis 0,9 Kallikrein inaktivierende Einheiten (weiter unten KIE genannt), Rest war Luft. Im Anwendungsfall eines pneumatischen Generators belief sich die Fördergeschwindigkeit des zu versprühenden Aerosolgemisches auf 9 l/min. Die Konzentration von Aprotinin im Gasgemisch betrug 0,09 bis 0,9 KIE/ml. Um die Aerosolstabilität zu erhöhen und die Adsorption von Aerosol im Respirationstrakt zu verbessern, wurden in eine zu versprühende Lösung NaCl, Glyzerin und eine ioneninaktive oberflächenaktive Substanz (Twin-80, Troton X-100 od. dgl.) eingegeben. Die zu testenden Aerosole wiesen bei einer pneumatischen Versprühung eine Dispersität der Teilchen von 1,9 µm (95 Masse-%) auf, bei einer Ultraschall-Versprühung hatten die Teilchen eine Größe von bis 100 µm, wobei 90 % der Teilchen bis 5 µm groß waren. Die an Kindern durchgeführten anfänglichen klinischen Untersuchungen haben ergeben, dass die Inhalation bei Anwendung eines Gas-Wasser-Gemisches von Aprotinin und das Einträufeln von Nasentropfen zu einer Verkürzung der Dauer von Krankheitsanzeichen (wie Schnupfen, Husten, Katarrh der oberen Luftwege) verhalf und die Entstehung wiederholter Komplikationen verhütete.

Die von dem Urheber beschriebenen Inhalationssysteme sind für die Erzeugung von Aerosolen nicht geeignet, welche an die Anwendung in Form von gasdichten Aerosoldruckbehältern mit normierter Dosierung und an die individuelle Behandlung eines Patienten gegen respiratorische Virusinfektionen angepasst sind. Dafür ist es notwendig, eine konkrete Zubereitung und eine einschlägige Technologie für die Befüllung von Behältern zu entwickeln. Aus diesem Grunde ist es unmöglich, den Wirkstoff einer Aerosolzubereitung auf dessen Stabilität gegen die Zerstörung und den Abbau des Wirkstoffes als Bestandteiles der Aerosolzubereitung zu prüfen.

### Darstellung der Erfindung

Aufgabe der Erfindung ist es, ein Aerosolpräparat auf Grundlage von Aprotinin bereitzustellen, das an die Anwendung in einem gasdichten Behälter mit normierter Dosierung unter Beibehaltung von Antiviruseigenschaften des Aerosols bei dauernder Lagerung angepaßt ist.

Das technische Ergebnis ist es, die nativen Antiprotease- und Antiviruseigenschaften von Aprotinin in einem Aerosolpräparat bei der Verwendung und einer dauernden Lagerung zu Lasten der betreffenden Wahl dessen Bestandteile und der einschlägigen Technologie für die Befüllung von Behältern konstant zu halten.

Die genannte Aufgabe wird dadurch gelöst, dass erfindungsgemäß ein Aerosolpräparat mit Aprotinin als Arzneimittel in einer Menge zwischen 23 und 30 mg pro 100 ml Präparat, 70 bis 84 Vol.-% 1,1,1,2-Tetrafluorethan als Treibgas, 5 bis 10 Vol.-% Glyzerin, Wasser als Lösungsmittel und einem Stabilisierungsmittel vorgeschlagen wird, wobei das Präparat an die Verwendung unter Druck in einem Aerosolbehälter mit einem Dosierventil angepasst ist. Dafür haben die Urheber Bestandteile gewählt, die miteinander kompatibel sind, eine Lösung mit einer therapeutisch wirksamen Menge an Aprotinin bilden und an die Verwendung in einem gasdichten Aerosolbehälter angepasst sind.

Für das Aerosolpräparat haben die Urheber in der Eigenschaft eines Arzneimittels eine wässrige konzentrierte Lösung von Aprotinin, also ein Proteaseinhibitor mit weitem Wirkungsspektrums, verwendet, welches Aprotinin aus den Lungen eines Rindviehs ausgeschieden wurde (Produkt der Fa. Pentapharm, Schweiz). Diese Firma ist der Lieferant von Aprotinin für die Gesellschaft GERMED (Deutschland), die ihrerseits das Aprotinin hat eingetragen lassen und das Aprotinin nach Russland unter der Handelsmarke KONTRIKAL liefert. Die von den Urhebern festgelegte therapeutisch wirksame Menge an Arzneimittel sorgt für die Wirksamkeit von Aprotinin bei Anwendung einer einzigen Dosis des Aerosolpräparates mit 85 bis 110 KIE.

Der Gehalt des Aerosolpräparates an 70 bis 84 Vol.-% des Treibgases bewirkt eine Kompatibilität des Arzneimittels mit restlichen Bestandteilen in Form einer Lösung in einer Aerosolpackung.

Die im Aerosolpräparat enthaltenen Lösungsmittel, nämlich 8 bis 15 Vol.-% Ethanol, 5 bis 10 Vol. % Glyzerin und Wasser, dessen Menge bis auf 100 ml Präparat Rest ist und sich auf bis 4 Vol.-% beläuft, dienen für eine Verbesserung der Löslichkeit von Aprotinin, wobei deren Menge die maximale Menge an Aprotinin definiert, die sich bei einer bestimmten Temperatur auflösen kann. Vorzugsweise benutzt man Äthanol als Lösungsmittel, in welchem eine geringe Menge an Wasser enthalten ist, wie z. B. 96%iges Äthanol, so dass es in azeotropischer Weise unter Bedingungen einer Temperatur und Feuchtigkeit verdampft.

Das Vorhandensein von Glyzerin in einer wünschenswerten Konzentration von 96 % im Präparat schützt die Aerosoltropfen vor schneller Trocknung und verbessert deren Adsorption an der Schleimhaut des Respirationstraktes. Ätherisches Öl, wie z. B. Pfefferminzöl, kommt als Konservierungsmittel (antimikrobielles Stabilisierungsmittel) in Frage, das dank den darin enthaltenen Phenolverbindungen zusätzlich eine bakterizide Wirksamkeit aufweist und eine Agglomeration von Teilchen des Präparates verhindert. Es ist wünschenswert, das Öl in einer Menge zwischen 0,6 und 1,0 Vol. % zuzugeben.

Als Verpackung für ein Heilmittelpräparat auf Basis von Aprotinin benutzt man einen Alu-Behälter, der in der Lage ist, dem Druck des darin befindlichen Treibgases standzuhalten und der mit einem Dosierventil versehen ist, das bei einer einzigen Verabreichung in die Lungen oder die Nase eines Patienten eine therapeutisch wirksame Menge an Aprotinin sicherstellt. Das Dosierventil kann eine 250 bis 460 µm weite Öffnung zum Auslaß des Aerosols besitzen, deren Weite sich normalerweise nach der Konzentration des Lösungsmittels und dessen Schnelligkeit des Verdampfes nach der Inhalation richtet.

### Beste Ausführungsform der Erfindung

Durch die Urheber wurde ein Aerosolbehälter (weiter unten Inhalator genannt) getestet, bei welchem eine Aprotininlösung durch eine 30 µm weite Öffnung herausgeblasen wird. Die Rolle einer Auftriebskraft spielt hier das Treibgas 1,1,1,2,-Tetrafluorethan (Treibgas 134 A) mit darin aufgelöstem Aprotinin. Flüssiges Gemisch aus dem Treibgas und der Aprotininlösung wird durch das Treibgas aus dem Behälter über ein Dosierventil herausgestoßen und in kleinsten Tropfen versprüht, die eine Aerosolwolke bilden. Das erfindungsgemäße Aerosolpräparat zählt zu einem feuchten Typ. Die Größe von Aerosoltropfen hängt von drei hauptsächlichen Faktoren ab, nämlich von der Auftriebskraft beim Austreten der Lösung aus dem Inhalator, dem Verhältnis Treibgas/Wirkstofflösung und von der Weite der Auslaßöffnung im Ventil. Praktisch sofort nach dem Austritt und der Zerstreuung von Aerosoltropfen verdampft das Treibgas und der Wirkstoff, also Aprotinin, bleibt in der restlichen Wasser-Glyzerin-Phase erhalten. Bei dem Aerosolbehälter sind das Verhältnis Treibgas/Wasserphase und die Ventilöffnung ausgewählt, derart, dass die Teilchengröße in einem Bereich zwischen 2 und 150 µm gesichert wird. Bei dem Profil hinsichtlich der Dispersität von Aerosol beträgt der Gewichtsanteil von Teilchen in einer Wolke mit den Abmessungen von 2 bis 15 µm (kleine Bronchien und Alveolen), von 15 bis 50 µm (größere und mittlere Bronchien, Kehlkopf, Mundrachenraum) und 50 bis 150 µm (Nasenrachenraum und Mundrachenraum) jeweilig 15, 60 und 25 %. (Dies stimmt gut mit Angaben aus der Literatur überein : Newman S., Pitcaim G., Steed K. et al., Deposition of fenoterol from pressurized metered dose inhalers containing hydrofluoroalkanes. J. Allergy Clin. Immunol., v. 104, p. 253 bis 257, 1999). Dieser Bereich der Dispersität von Aerosolteilchen des Aprotinins ist zielbewusst für die Behandlung der Grippe und von anderen akuten respiratorischen Virusinfektionen gedacht.

Es ist gut bekannt, dass bei Erkrankungen des Respirationstraktes, insbesondere in einer frühzeitigen Phase des Prozesses, die Infektion im Nasen- und dem Mundrachenraum lokalisiert wird, wobei im Falle einer fortschreitenden Entfaltung der Infektionsprozess in die unteren Abteilungen wie Kehlkopf, Luftröhre und mittlere Bronchien sinken kann. Und nur bei schweren Fällen erstreckt sich die Infektion auf distale Abteilungen wie Bronchiolen, Alveolarzone. Eine Analyse des Profils der Dispersität von Aerosol hat gezeigt, dass die Verwendung des erfindungsgemäßen Aerosolpräparates im Wesentlichen für die oberen und mittleren Abteilungen des Respirationstraktes wirksam ist, in welche Teilchen mit einem Durchmesser zwischen 5 und 100 µm eindringen. Ausgerechnet dieses Profil benötigt man bei der individuellen Verwendung in ursprünglichen Etappen der Entstehung einer respiratorischen Viruskrankheit und einer frühzeitigen Prophylaxe beispielsweise in Familien.

Die Urheber haben ein biomedizinisches Testen des beanspruchten Aerosolpräparates im D.I. Iwanowski-Forschungsinstitut für Virusologie der Russischen Akademie der medizinischen Wissenschaften und eine Prüfung auf die strukturelle und funktionale Bioidentität durch die Gesellschaft WLK Ltd. (Tschechien) durchführen lassen. Besonders ausschlaggebend ist dabei die Frage von der Konstanthaltung der Sekundärstruktur und der Wirksamkeit von Aprotinin im Aerosolbehälter, nachdem es diesen verlassen hat. Dem Testen wurde eine Lösung ausgesetzt, die übriggeblieben war, nachdem die flüchtigen Bestandteile wie Treibgas und z. T. Äthylalkohol den Inhalator verlassen haben und naturgemäß verdampft sind.

Eine wichtige Bedeutung dieses Testes gipfelte in einem Nachweis für die biologische Wirksamkeit von Aprotinin nach dessen Auflösen im Treibgas-System und dem Versprühen der hergestellten Lösung mittels des Inhalators. Beim Testen wurde festgestellt, dass das Aprotinin, das in einem Alkohol-Glyzerin-System des Treibgases 134A aufgelöst war, die Löslichkeit beibehielt und nicht ausfiel;
dass das Aprotinin, das in einem Alkohol-Glyzerin-System des Treibgases 134A aufgelöst war, seine ursprünglichen Eigenschaften innerhalb von mindestens 3 Jahren stabil beibehielt ;
dass das Aprotinin, das in einem Alkohol-Glyzerin-System des Treibgases 134A aufgelöst war, seine nativen Struktureigenschaften beibehielt;
dass das Aprotinin, das in einem Alkohol-Glyzerin-System des Treibgases 134A aufgelöst war, seine Antiprotease-Wirksamkeit beibehielt und
dass das Aprotinin, das in einem Alkohol-Glyzerin-System des Treibgases 134A aufgelöst war, seine Antiviruseigenschaften beibehielt.

Es ist wichtig hervorzuheben, dass die gesamte Palette an Eigenschaften von Aprotinin auch nach dessen Austritt aus dem Inhalator erhalten bleibt.

Somit hat dieser Test nachgewiesen, dass, erstens, das Alkohol-Glyzerin-System des Treibgases 134A mit dem Stoff der Polypeptidnatur, also dem Aprotinin, kompatibel ist und keine dessen Denaturierung und Inaktivierung hervorruft und dass, zweitens, das im Aerosolbehälter befindliche Präparat ein aus wirksamem Aprotinin bestehendes Aerosol unter Beibehaltung von dessen nativen Antiprotease-und Antiviruseigenschaften im Aerosol bildet. Um die Stabilität des Präparates bestimmen zu können, wurden Aerosolbehälter an einer trockenen, lichtgeschützten Stelle bei einer Temperatur von 25 °C gelagert, wobei eine Packung 250 Dosen enthielt. Die Ergebnisse der Untersuchung auf die Stabilität des Heilmittelpräparates sind nachfolgend in der Tabelle 1 zusammengefasst.

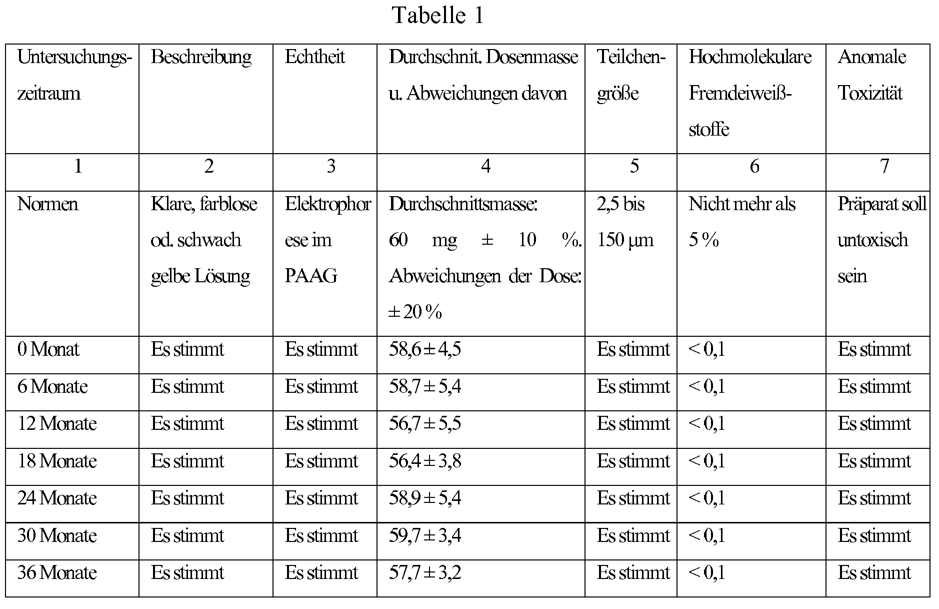

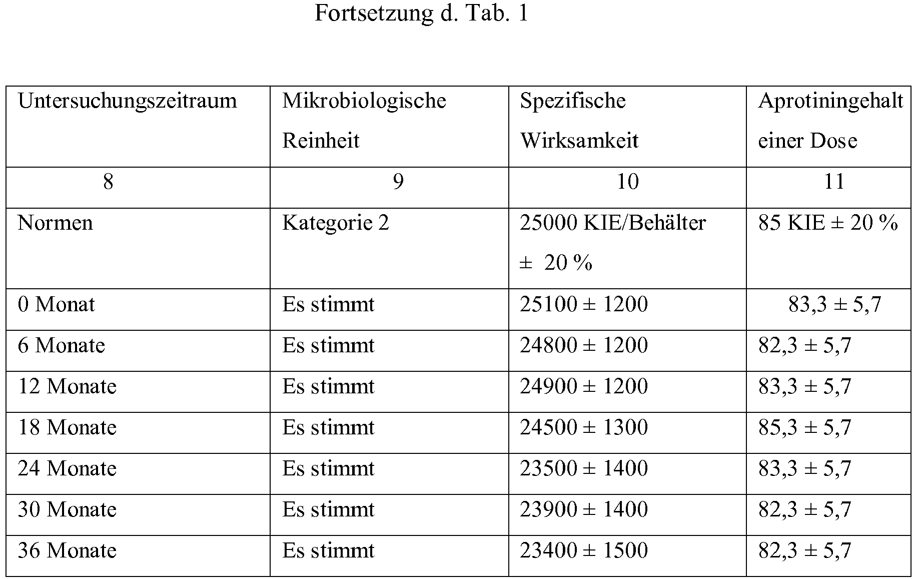

Klinische Untersuchungen des Präparates wurden in der Herbst-Winterzeit an Patienten durchgeführt, die an akuten Krankheiten des Respirationstraktes (überwiegend der oberen Abteilungen wie Nasenrachenraum, Bronchien) litten, die durch Viren von Grippe, Paragrippe, durch Adenoviren und eine gemischte, durch die genannten Viren hervorgerufene Infektion verursacht waren. Der größte Teil der Kranken hatte eine mittlere Schwere des Krankheitsverlaufes. An den Untersuchungen beteiligten sich 179 Patienten im Alter von 27 bis 65 Jahren. Die Patienten genossen eine symptomatische Therapie unter Anwendung üblicher Mittel wie Sodainhalation, Vitamine, Analgetika, Beruhigungsmittel (Placebo-Gruppe). Statt der Sodainhalationen verwendete man in der Eigenschaft eines pathogenetischen Antivirusmittels ein erfindungsgemäßes Aerosolpräparat Aprotinin (Versuchsgruppe), das unter Druck in gasdichte Aerosolbehälter eingebracht war.

Vor dem Anfang und am Ende der Kur bestimmte man den Bilirubingehalt des Blutes, die Aktivität einer Reihe von Enzymen (Aspartaminotransferase, Alaninaminotransferase), Kennwerte einer Thymolprobe, den Gehalt des Blutes an Hämoglobin, weißen Blutkörperchen, Neutrophilen, Lymphozyten, Monozyten und die Blutsenkungsgeschwindigkeit. Diese Untersuchungen gestatteten es, eine mögliche allgemeine toxische Wirkung des Präparates zu beurteilen.

Aprotinindosen, die den Patienten bei der Handhabung eines Inhalators verabreicht wurden, reichten für 2 bis 4 Aerosoleinspritzungen in die Nase oder den Mund alle 2 bis 3 Stunden aus, wobei sich die rechnerisch ermittelte Tagesdosis auf 300 bis 500 KIE/Tag je Patient entsprechend dem Modus für die klinischen Untersuchungen belief. Auf diese Art und Weise besorgte das erfindungsgemäße Aerosolpräparat die therapeutische Basiskur im Bereich der empfohlenen Dosen, die ausgehend von den Ergebnissen der klinischen Untersuchungen festgelegt waren.

Für alle Kranken nach der Aprotinininhalation wurde ein guter therapeutischer Effekt festgestellt. Dank der Verwendung von Aprotinininhalationen konnte die Genesung der Kranken mit akuten respiratorischen Virusinfektionen beschleunigt und die Gefahr von bakteriellen Komplikationen herabgesenkt werden. Insbesondere normalisierte sich die Temperatur bei Kranken, denen Aprotinin verabreicht wurde, im Durchschnitt nach Ablauf von 2,68 Tagen und bei Kontrollkranken mit einer genormten Behandlung ohne Verabreichung von Aprotinin erst nach Ablauf von 4,05 Tagen (p < 0,05). Bei Verabreichung von Aprotinin hörten die Symptome einer Intoxikation bereits nach 3 Tagen auf, während bei der Gruppe mit Placebo diese Symptome innerhalb von 4,14 Tagen (p< 0,05) feststellbar waren. Die Vorteile der Behandlung mit dem Aprotinin kamen durch eine Verkürzung der Dauer des katarrhalischen Syndroms zutage. Insbesondere betrug die Dauer des Schnupfens bei Verabreichung von Aprotinin 2,56 Tage und bei Verabreichung von Placebo 4,05 Tage (p < 0,05), die Dauer der Halsschmerzen betrug 2,27 Tage, bei Verabreichung von Placebo 3,17 Tage (p <0,05), die Dauer eines trockenen Hustens betrug bei Verabreichung von Aprotinin 2,5 Tage und bei Verabreichung von Placebo 5,5 Tage (p < 0,05). Topische Entzündungssymptome im Mundrachenraum und den oberen Luftwegen verschwanden bei Verabreichung von Aprotinin im Durchschnitt nach 5,08 Tagen und in der Placebo-Gruppe nach 6,82 Tagen (p < 0,05). Die Hyperämie der Schleimhaut im Mundrachenraum hörte vor dem Hintergrund der Behandlung mit Aprotinin nach Ablauf von 5 Tagen und bei der Kontrollkranken erst nach Ablauf von 6,82 Tagen (p < 0,05) auf. Vergrößerte Mandeln und eine Körnung der Hinterwand des Rachens verschwanden bei der Behandlung mit Aprotinin schneller als bei der Behandlung von Kranken ohne Verabreichung von Aprotinin.

Zusammenfassend kann man ausgehend von den gewonnenen Angaben einen Schluß ableiten, dass die Behandlung von akuten respiratorischen Virusinfektionen unter Verwendung von Aprotinininhalationen die Genesung beachtlich beschleunigt, was darin begründet liegt, dass sich die Dauer hauptsächlicher Manifestationen einer akuten respiratorischen Virusinfektion um 26 bis 55 % gegenüber der genormten Behandlung verkürzt. Bei Nasalabspülungen ließ sich der Grippevirus vor dem Hintergrund der Behandlung mit Aprotinin bereits nach Ablauf von 2 bis 3 Tagen bezogen auf den Anfang der Behandlung nicht bestimmen. In vier Fällen traten bei Patienten mit Verabreichung von Placebo bakterielle Komplikationen in Form einer Bronchitis und einer Pneumanie auf, so dass man auf eine zusätzliche Behandlung mit antibakteriellen Präparaten angewiesen war. Weder toxische Wirkung noch Reizwirkung durch die Aprotinininhalation wurde bei keinem der behandelten Patienten innerhalb der gesamten Versuchsdauer festgestellt. Nachfolgend sind Verfahren zur Herstellung eines erfindungsgemäßen Aerosolpräparates aufgeführt.

### Beispiele

Die Zusammensetzungen von drei Aprotininaerosolen in Form einer erfindungsgemäßen Lösung sind in Tabelle 2 zusammengefasst.

**Tabelle 2**

| Aerosolbestandteile | Zusammensetzung eines Präparates in einer Menge von 100 ml | | | |
|---|---|---|---|---|
| Aprotinin, mg | 23 | 30 | 23 | 25 |
| 1,1,1,2-Tentafluorethan, ml | 84 | 70 | 79 | 80 |
| Äthanol, mg | 8 | 15 | 13 | 10 |
| Glyzerol, ml | 5 | 10 | 6,0 | 7,2 |
| Pfefferminzöl, ml | 0,6 | 1 | 0,79 | 0,8 |
| Wasser, ml | 2,4 | 4 | 1,21 | 2 |

Aerosole wurden durch das Mischen von Bestandteilen bei folgenden Schritten hergestellt :
1. Abwägen und Mischen von Lösungsmitteln und einer konzentrierten wässrigen Aprotininlösung (Basismischung) ;
2. Abfüllung der Basismischung in einen Alu-Behälter ;
3. Abdrücken des Ventils des Behälters ;
4. Zusetzen einer bekannten Menge an Treibgas in den Behälter über das Ventil unter Druck, wobei dies nur bei gasdichter Anbringung des Ventils zulässig ist.

Die fertigen Behälter werden automatisch beschriftet und nach der Ausstattung mit Mund- und Nasenansatzstücken aus Kunststoff in einen Karton zum Versand an Verbraucher eingepackt.

Dabei handelt es sich um einen Alu-Behälter (Inhalator) mit einer normierten Dosierung von 350 Dosen und einem Fassungsvermögen von 30 ml oder von 250 Dosen und einem Fassungsvermögen von 20 ml.

Die Eigenschaften des Aerosolaprotinins ermittelte man ausgehend von einem oder mehreren folgenden Merkmalen :
- Aussehen (es durften außen keine Merkmale einer Undichtigkeit oder Deformation vorhanden sein) ;
- Behälterinhalt, welcher innerhalb von 10 % bezogen auf den Mittelwert liegen soll ;
- Menge an Arzneimittel je Behälter, die innerhalb von 20 % bezogen auf den Mittelwert liegen soll ;
- Anteil des ausgetretenen Behältergehaltes beträgt mindestens 75 % ;
- Abweichung der normierten Dosis von der mittleren Masse liegt innerhalb von 20 %.

Das Präparat soll bei der Behandlung von respiratorischen Virusinfektionen eine breite Verwendung finden.

## Patentansprüche

1. Aerosolpräparat zur Behandlung von respiratorischen Virusinfektionen, enthaltend Aprotinin als Wirkstoff in einer Menge zwischen 23 und 30 mg pro 100 ml des Präparates, 70 bis 84 Vol.-% 1,1,1,2-Tetrafluorethan als Treibgas, 8 bis 15 Vol.-% Äthanol als Lösungsmittel, 5 bis 10 Vol.-% Glyzerol, Wasser und ein antimikrobielles Stabilisierungsmittel, wobei es an die Verwendung unter Druck in einem Aerosolbehälter mit einem Dosierventil angepasst ist.

2. Aerosolpräparat nach Anspruch 1, **dadurch gekennzeichnet, dass** das genannte Lösungsmittel 96 Vol.-% Äthanol aufweist.

3. Aerosolpräparat nach Anspruch 1, **dadurch gekennzeichnet, dass** das genannte Lösungsmittel 96 Vol.-% Glyzerin aufweist.

4. Aerosolpräparat nach Anspruch 1, **dadurch gekennzeichnet, dass** die mittlere Teilchengröße von Aerosol von 2,5 µm bis 150 µm beträgt.

5. Aerosolpräparat nach Anspruch 1, **dadurch gekennzeichnet, dass** es Pfefferminzöl als Stabilisierungsmittel in einer Menge zwischen 0,6 und 1,0 Vol.-% aufweist.

6. Aerosolpräparat nach Anspruch 1, **dadurch gekennzeichnet, dass** es Wasser in einer Menge von bis 4 Vol.-% aufweist.

7. Aerosolpräparat nach Anspruch 1, **dadurch gekennzeichnet, dass** der Dispersionsgrad von Teilchen des Aprotinaerosols zwischen 2 µm und 150 µm beträgt.

## Claims

1. Aerosol preparation for the treatment of respiratory viral infections, containing aprotinin as active substance in an amount of 23 and 30 mg per 100 ml of the preparation, 70 to 84 vol% 1,1,1,2-tetrafluoroethane as propellant, 8 to 15 vol% ethanol as solvent, 5 to 10 vol% glycerol, water and a stabilizer, said it being adapted for use under pressure in an aerosol preparation with a metering valve.

2. Aerosol preparation as defined in claim 1, wherein the specified solvent contains 96 Vol% ethanol.

3. Aerosol preparation as defined in claim 1, wherein the specified solvents contains 96 Vol% glycerol.

4. Aerosol preparation as defined in claim 1, wherein the average particle size of the aerosol is between 2.5 µm and 150 µm.

5. Aerosol preparation as defined in claim 1, wherein peppermint oil is utilized as stabilizer in an amount of 0.6 to 1.0 Vol%.

6. Aerosol preparation as defined in claim 1, wherein it contains water in an amount of up to 4 Vol%.

7. Aerosol preparation as defined in claim 1, wherein the degree of dispersion of the particles of the aprotinin aerosol is between 2 µm and 150 µm.

## Revendications

1. Préparation d'aérosol pour le traitement d'infections respiratoires virales, contenant de l'aprotinine en tant que principe actif dans une quantité comprise entre 23 et 30 mg par 100 ml de préparation, de 70 à 84 % en volume de 1,1,1,2-tétrafluoroéthane en tant que gaz propulseur, de 8 à 15 % en volume d'éthanol en tant que solvant, de 5 à 10 % en volume de glycérol, de l'eau et un stabilisant antimicrobien, la préparation d'aérosol étant adaptée à l'utilisation sous pression dans un conteneur d'aérosol comportant une valve de dosage.

2. Préparation d'aérosol selon la revendication 1, **caractérisée en ce que** ledit solvant présente 96 % en volume d'éthanol.

3. Préparation d'aérosol selon la revendication 1, **caractérisée en ce que** ledit solvant présente 96 % en volume de glycérine.

4. Préparation d'aérosol selon la revendication 1, **caractérisée en ce que** la taille de particules moyenne de l'aérosol est de 2,5 µm à 150 µm.

5. Préparation d'aérosol selon la revendication 1, **caractérisée en ce qu'**elle présente de l'huile essentielle de menthe poivrée en tant que stabilisant dans une quantité comprise entre 0,6 et 1,0 % en volume.

6. Préparation d'aérosol selon la revendication 1, **caractérisée en ce qu'**elle présente de l'eau dans une quantité allant jusqu'à 4 % en volume.

7. Préparation d'aérosol selon la revendication 1, **caractérisée en ce que** le degré de dispersion des particules de l'aérosol d'aprotinine est compris entre 2 µm et 150 µm.
